# FASCICULE DE BREVET EUROPEEN

(11) **EP 0 785 769 B1**
(45) Date de publication et mention de la délivrance du brevet: **10.03.1999**
(21) Numéro de dépôt: 95927758.3
(22) Date de dépôt: 04.08.1995
(51) Int. Cl.: A61K 9/00

(54) **ASSOCIATION THERAPEUTIQUE VITAMINO-CALCIQUE SOUS FORME GALENIQUE UNITAIRE DE COMPRIMES, SON PROCEDE D'OBTENTION ET SON UTILISATION**
THERAPEUTISCHE MISCHUNG AUS VITAMIN UND CALCIUM IN FORM EINER GALENISCHEN EINZELDOSISTABLETTE, VERFAHREN ZU IHRER HERSTELLUNG UND IHRE VERWENDUNG
THERAPEUTIC VITAMIN-CALCIUM COMBINATION IN UNITARY GALENIC TABLET FORM, METHOD FOR PREPARING SAME AND USE THEREOF

(30) Priorité: 23.09.1994 FR 9411381
(43) Date de publication de la demande: 30.07.1997
(73) Titulaire: LABORATOIRE INNOTHERA Société Anonyme, 94111 Arcueil (FR)
(72) Inventeur: MEIGNANT, Catherine, F-75010 Paris (FR); STENGER, Eric, F-94800 Villejuif (FR)
(74) Mandataire: Dupuis-Latour, Dominique
(86) Numéro de dépôt international: FR9501053
(87) Numéro de publication internationale: WO9609036

(56) Documents cités:
- EP-A- 0 413 828
- Physician's Desk Reference, PDR 14th ed., 1993, OS-CAL 500 + D Tablets
- Physician's Desk Reference, PDR 46th ed., 1992, DICAL-D Tablets
- Physician's Desk Reference, PDR 1993, 14th ed., p. 576
- VIDAL 1994, p. CALCIPRAT 750 mg

## Description

La présente invention concerne une nouvelle association thérapeutique vitamino-calcique, son procédé d'obtention et son utilisation.

On connaît de nombreuses associations vitamino-calciques pour combattre diverses maladies.

Les effets thérapeutiques liés à l'administration conjointe de calcium et de vitamine D sont bien connus, comme cela est relaté par exemple dans les articles de Marie C. Chapuy et coll. ― Effect of Calcium and Cholecalciferol Treatment for Three Years on Hip Fractures in Elderly Women, *British Medical Journal*, 308, 1081-1082 (23 avril 1994), de Marie C. Chapuy et coll. ― Vitamin D3 and Calcium to Prevent Hip Fractures in Elderly Women, *New England Journal of Medicine*, 327, 1637-1642 (3 décembre 1992) et dans l'article intitulé Supplementation with Vitamin D3 and Calcium Prevents Hip Fractures in Elderly Women, *Nutrition Reviews*, Vol. 51, 6, pp. 183-185.

Ces articles montrent également la variabilité des effets thérapeutiques de l'association en fonction du dosage de calcium et de la vitamine D, avec une dose journalière optimale se situant, pour une indication dans la prévention et le traitement de l'ostéoporose, aux alentours de 1000 à 1200 mg de calcium élément et 800 UI de vitamine D3.

Le calcium et la vitamine D sont généralement administrés au patient simultanément, mais sous des formes distinctes, par exemple des comprimés d'un sel de calcium et des gouttes de vitamine D.

En effet, les sels de calcium acceptables du point de vue pharmaceutique et la vitamine D présentent, chacun en ce qui les concerne, des caractéristiques très spécifiques du point de vue de la galénique (voir notamment le EP-A-0 413 828 qui concerne une préparation stabilisée de vitamine D3 destinée à potentialiser la stabilité du principe actif), ce qui conduit à un conditionnement sous des formes séparées.

Mais ceci rend difficile le respect des doses absolues et relatives de calcium et de vitamine D, et donc l'observance correcte du traitement, en particulier sur une longue période.

Il a été déjà proposé des associations calcium et vitamine D sous une même forme, par exemple par le WO-A-94 06435 (procédé de traitement gynécologique utilisant notamment une combinaison de vitamine D et de calcium), le WO-A-92 19251 (association de vitamine D avec du calcium pour combattre l'ostéoporose, plus particulièrement sous forme buvable), le EP-A-0 197 514 (composition pharmaceutique comprenant une hormone parathyroïde ou un fragment physiologiquement actif de celle-ci en combinaison avec de la vitamine D hydroxylée ou un sel de calcium non toxique pour augmenter la masse osseuse) ou encore le DE-A-42 12 122 (élément basse calorie à base de protéines, d'un sel de calcium et de vitamine D).

Mais dans ces formes connues les proportions entre calcium et vitamine D sont généralement assez éloignées des proportions optimales souhaitables indiquées notamment dans la littérature précitée.

Ces formes connues correspondent d'ailleurs souvent plus à des suppléments vitamino-calciques (suppléments alimentaires ou spécialités "OTC" vendues sans prescription médicale) qu'à de véritables spécialités pharmaceutiques à visée thérapeutique destinées à prévenir ou traiter des affections telles que l'ostéoporose avec une posologie précise.

Il existe ainsi à l'heure actuelle un besoin de pouvoir disposer d'une association vitamino-calcique comportant, sous une seule et même forme de comprimé à croquer, un dosage relatif optimal entre calcium et vitamine D, tout particulièrement pour la prévention et le traitement de l'ostéoporose.

Mais du fait de la nature des sels de calcium disponibles acceptables du point de vue pharmaceutique, il est relativement difficile d'associer du calcium avec de la vitamine D dans certains dosages spécifiques. Ceci est particulièrement vrai si l'on désire obtenir des comprimés par un procédé de fabrication par compression directe. Les contraintes des principes actifs, à savoir le calcium et la forme de vitamine D, ne permettent alors pas une mise en oeuvre directe.

La présente invention résout les problèmes mentionnés ci-dessus en proposant une association thérapeutique vitamino-calcique selon la revendication 1, et un procédé de fabrication selon la revendication 13. Les sous-revendications visent des caractéristiques subsidiaires avantageuses.

Divers avantages et caractéristiques de la présente invention ressortiront d'un exemple de réalisation ci-après.

Dans cet exemple, l'association de l'invention se présente sous forme d'un comprimé à croquer de formule suivante (pour un comprimé terminé à 2500 mg) :

| | |
|---|---|
| Calcium (carbonate de) | 1 250 mg |
| (Quantité correspondant à calcium élément | 500 mg) |
| Cholécalciférol | 4 mg* |
| Xylitol | 661 mg |
| Sorbitol | 500 mg |
| Polyvinylpyrrolidone | 45 mg |
| Arôme (citron, orange, etc.) | 20 mg |
| Stéarate de magnésium | 20 mg |

| | |
|---|---|
| (* Vitamine D3 dosée à 100 000 UI/g) | |

Le carbonate de calcium est du type SCORALITE 1B®, SCORA ; il s'agit d'une poudre blanche de granulométrie très fine d'un diamètre moyen de 12 micromètres environ, de densité élevée (d = 1,3 g/cm³ environ) présentant un mauvais écoulement et une mauvaise aptitude à la compression.

La vitamine D est du cholécalciférol (type 100 CWS®, ROCHE) ; il s'agit d'une poudre granuleuse, de diamètre moyen de 200 micromètres environ, de couleur jaunâtre, dosée à 100 000 UI par gramme.

La présence de DL-α-tocophérol (environ 0,2 % m/m de vitamine E) lui confère une grande stabilité et empêche son oxydation.

Le diluant-édulcorant utilisé dans l'invention est de préférence du xylitol de type XYLITAB 300®, FINNSUGAR. Ce xylitol est un polyol de saveur sucrée (équivalente à celle du saccharose), procurant une agréable sensation de fraîcheur dans la bouche, il est acariogène et très peu calorique (2,4 Kcal/g contre 4 Kcal/g pour le saccharose). Cette sensation agréable permet une meilleure observance du traitement par le patient. Ce xylitol utilisé possède des propriétés de comprimabilité supérieures à celles du xylitol standard.

Ce composé se présente sous la forme d'une poudre granuleuse, cristalline blanche, d'un diamètre moyen de 250 micromètres.

Le liant-édulcorant utilisé dans la présente invention est en particulier du sorbitol (de type NEOSORB P 60 W®, ROQUETTE). Ce polyol se présente sous la forme d'une poudre granuleuse blanche, d'un diamètre moyen de 200 micromètres et possède d'excellentes propriétés liantes en compression. Le sorbitol est de saveur sucrée (70 % de celle du saccharose), acariogène et peu calorique (2,4 Kcal/g).

Le liant de la présente invention est, de préférence, de la polyvinylpyrrolidone (de type KOLLIDON K 30®, BASF) ; il se présente sous la forme d'une poudre blanchâtre granuleuse et possède de très grandes propriétés liantes en granulation humide. La valeur de la constante K caractérise les polyvinylpyrrolidones solubles et dépend de leur solubilité relative.

L'aromatisant est particulièrement un arôme citron (SBI) ; il se présente sous forme d'une poudre fine, jaunâtre, composée d'huiles essentielles atomisées sur de la maltodextrine. De nombreux essais réalisés durant la mise en oeuvre de la présente invention qui ont comparé différents arômes ont montré que l'arôme citron convenait parfaitement bien au masquage du goût crayeux du carbonate de calcium et qu'il s'associait agréablement à la sensation de fraîcheur apportée par le xylitol.

Le lubrifiant est généralement du stéarate de magnésium se présentant sous forme d'une poudre fine, blanchâtre, permettant d'éviter le phénomène de grippage au niveau des matrices des presses à comprimer quand l'association vitamino-calcique de la présente invention est sous forme de comprimés.

La quantité de calcium élémentaire par prise sera, de préférence, de 500 mg, ce qui correspond à 1250 mg de carbonate de calcium.

La quantité de cholécalciférol est de 4 mg par prise, ce qui correspond à 400 UI d'une vitamine D3 dosée à 100 000 UI/g. En pratique, la quantité de cholécalciférol par comprimé dépend du dosage de la matière première utilisée.

Ces doses correspondent notamment à la posologie optimale indiquée par les publications mentionnées plus haut, tant en valeur absolue (doses journalières de calcium et de vitamine D3, respectivement) que relative (ratio calcium/vitamine de l'ordre de 1,25 mg de Ca élément par UI de vitamine D).

Les nombreux essais des formules de cet exemple ont permis d'optimiser les quantités des différents excipients.

Pour obtenir un comprimé à croquer au goût le plus agréable, l'apport en liant à sec et en milieu humide combiné en quantité synergique avec au moins le diluant édulcorant, au moins le liant édulcorant et au moins le lubrifiant doit être important. Dans le cas d'un comprimé, celui-ci aura généralement une masse de 2500 mg.

Dans certaines formes de mise en oeuvre de la présente invention, on utilise une quantité de xylitol d'environ 661 mg, qui correspond à la quantité nécessaire à incorporer pour obtenir le meilleur masquage de goût du carbonate de calcium sans pour autant diminuer la comprimabilité du mélange, les propriétés du xylitol en compression étant moyennes.

Le sorbitol est utilisé à raison d'environ 500 mg car il s'agit de la quantité nécessaire à incorporer pour obtenir une parfaite reproductibilité de la fourchette de résistance à la rupture, paramètre critique dans le cas des comprimés à croquer. Une quantité supérieure, au détriment du xylitol, diminuerait les qualités gustatives du comprimé.

La polyvinylpyrrolidone est utilisée à raison d'environ 45 mg, lors de la granulation humide du carbonate de calcium, une partie (20 mg) est mélangée à sec avec le carbonate de calcium, la partie restante (25 mg) est utilisée en solution à 10 % dans de l'eau déminéralisée à froid. Une teneur en polyvinylpyrrolidone inférieure à 40 mg entraîne une trop grande friabilité des grains de carbonate de calcium. Une quantité plus importante n'apporte pas de réels bénéfices.

La quantité d'arôme citron est d'environ 20 mg, il s'agit de la quantité nécessaire pour aromatiser de façon satisfaisante le comprimé. Une faible variation de cette quantité (± 3 mg) ne modifie pratiquement pas le goût final.

La quantité de stéarate de magnésium est d'environ 20 mg. Il s'agit de la quantité nécessaire pour obtenir une lubrification satisfaisante lors de la compression. Une quantité plus faible, environ 15 mg, entraîne un phénomène de grippage, alors qu'une quantité supérieure, 25 mg tend à diminuer la dureté du comprimé et risque de modifier son goût.

Les caractéristiques physiques des éléments de l'association vitamino-calcique de la présente invention vont être indiquées ci-après.

Le carbonate de calcium a un écoulement nul et une densité apparente (g/cm³) d'environ 1,28 à 1,35 et une humidité résiduelle en pourcent de 0,1. La vitamine D3 cholécalciférol sous forme d'un concentrat de forme pulvérulente a un écoulement de 6 secondes pour 100 g de poudre, une densité apparente en g/cm³ de 0,73, une humidité résiduelle en pour-cent de 6,4 et un dosage en UI/g de 100 000.

Le xylitol a un écoulement nul, une densité apparente en g/cm³ d'environ 0,68-0,69, une humidité résiduelle en pour-cent de 0,2 à 0,3.

Le sorbitol a des écoulements dans la gamme de 4 à 5 secondes pour 100 g de poudre, une densité apparente en g/cm³ de 0,71 à 0,73 et une humidité résiduelle en pour-cent de 0,5 à 0,8.

Les étapes de mise en oeuvre préférées du procédé d'obtention de l'association vitamino-calcique de la présente invention vont maintenant être exposées.

Une granulation humide du carbonate de calcium est tout d'abord effectuée.

Dans cette mise en oeuvre, le carbonate de calcium et la polyvinylpyrrolidone sont tamisés sur un tamiseur vibrant pourvu d'une grille d'ouverture de maille appropriée ; ces poudres sont introduites dans un mélangeur et mélangées peu de temps à une vitesse appropriée. De la solution de polyvinylpyrrolidone est ajoutée par étapes successives. Une granulation est effectuée jusqu'à l'obtention d'une masse humide, permettant une étape de précalibrage suivante.

Une précalibration est effectuée sur un granulateur muni d'une grille d'ouverture de maille appropriée.

Le produit résultant est séché sur un lit d'air fluidisé et laissé à refroidir.

La perte de masse à la dessiccation est déterminée et une calibration sur une grille d'ouverture de maille appropriée est effectuée.

Parallèlement, la vitamine D3 est prémélangée et, après un tamisage, elle est mélangée avec du sorbitol dans un mélangeur pendant une durée appropriée et à une vitesse de rotation convenable.

On réalise ensuite le mélange avec les autres constituants en tamisant du xylitol, du sorbitol et l'arôme sur un tamis vibrant équipé d'une grille à ouverture de mailles appropriée. On mélange ces trois constituants avec le prémélange de vitamine D3-sorbitol dans un mélangeur à une vitesse appropriée. On introduit ensuite le carbonate de calcium granulé et on le mélange pendant la durée requise à la vitesse appropriée.

Le stéarate de magnésium est tamisé sur un tamis vibrant muni d'une grille d'ouverture de maille appropriée puis l'ensemble est mélangé dans un mélangeur.

L'ensemble ci-dessus peut être ensuite comprimé sur une presse à comprimer en contrôlant régulièrement l'uniformité de la masse et la résistance à la rupture. Les durées de mélange, les vitesses de rotation et les dimensions des tamis sont classiques et sont bien connues de l'homme du métier.

Ainsi, la présente invention permet d'obtenir une association vitamino-calcique contenant 500 mg de calcium élémentaire et 4 mg de vitamine D3 par prise, association qui est sous la forme d'un comprimé à croquer qui est d'un goût agréable et d'une dureté adaptée aux patients.

Plus particulièrement, à titre d'exemple non limitatif, les doses qui seront généralement utilisées se situeront dans les gammes suivantes : calcium sous forme élémentaire, environ 500 mg à environ 1500 mg ; vitamine D ou mélange de vitamines D, environ 3,5 mg à environ 12 mg. Une telle association vitamino-calcique sous forme de comprimés, ne contient ni sucre ni sodium.

De façon générale, pour la mise en oeuvre de comprimés du type à croquer ou à sucer on utilisera du carbonate de calcium. D'autres sels tels que le triphosphate de calcium peuvent être utilisés, mais leur absorption par l'organisme est moindre, ce qui conduit à adapter les quantités en conséquence (pour une même quantité de calcium élémentaire absorbé, il faut environ 1,2 g de triphosphate de calcium pour 1 g de carbonate de calcium).

## Revendications

1. Association thérapeutique vitamino-calcique sous forme galénique unitaire de comprimés, comprenant comme principes actifs associés du calcium et au moins une vitamine D, caractérisée en ce que, la forme galénique étant celle d'un comprimé à croquer, elle renferme, en outre, au moins un premier liant à sec et en milieu humide combiné en quantité synergique avec au moins un diluant, au moins un second liant et au moins un lubrifiant, l'un au moins dudit diluant et dudit second liant étant un édulcorant de type polyol, et en ce que le rapport du calcium à la vitamine D, exprimé en mg de Ca élément par UI de vitamine D, est compris entre 1 et 1,5.

2. Association selon la revendication 1, où le rapport du calcium sous forme élémentaire à la vitamine D, exprimé en mg de Ca élément par UI de vitamine D, est compris entre 1,2 et 1,3.

3. Association selon la revendication 1, où ledit diluant et ledit second liant sont tous deux des édulcorants de type polyol.

4. Association selon la revendication 1 ou 3, où le(s) polyol(s) est (sont) choisi(s) parmi le mannitol, le sorbitol, le xylitol et le maltitol.

5. Association selon la revendication 1, où le calcium provient d'un sel de calcium choisi parmi le carbonate de calcium, le pidolate de calcium, le lactate de calcium, le citrate de calcium, le gluconate de calcium, le chlorure de calcium, le glucoheptonate de calcium, le glycérophosphate de calcium et le phosphate de calcium.

6. Association selon la revendication 1, où la vitamine D est choisie parmi la vitamine D2 ou ergocalciférol, la vitamine D3 ou cholécalciférol ou un mélange de celles-ci.

7. Association selon la revendication 1, où le premier liant à sec et en milieu humide est choisi parmi une cellulose, la maltodextrine et la polyvinylpyrrolidone.

8. Association selon la revendication 1, où le lubrifiant est choisi parmi le stéarate de magnésium, l'acide stéarique, l'huile de ricin hydrogénée, l'huile de coton hydrogénée et le béhénate de glycérol.

9. Association selon la revendication 1, renfermant en outre un agent aromatisant.

10. Association selon la revendication 1, renfermant en outre un acidifiant.

11. Association selon la revendication 1, renfermant en outre un édulcorant supplémentaire choisi parmi le saccharinate de sodium, le cyclamate de sodium et l'aspartame.

12. Association selon les revendications 2 et 3 prises en combinaison, de formule générale :
| | |
|---|---|
| Calcium (carbonate de) | 1 250 mg |
| (Quantité correspondant à calcium élément | 500 mg) |
| Cholécalciférol | 4 mg * |
| Xylitol | 661 mg |
| Sorbitol | 500 mg |
| Polyvinylpyrrolidone | 45 mg |
| Arôme (citron, orange, etc.) | 20 mg |
| Stéarate de magnésium | 20 mg |
| | |
|---|---|
| (* Vitamine D3 dosée à 100 000 UI/g), ladite formule correspondant à un comprimé terminé à 2 500 mg. | |

13. Procédé d'obtention d'une association thérapeutique vitamino-calcique selon l'une des revendications 1 à 12, caractérisé en ce qu'il consiste :
(a) à granuler un sel de calcium avec un premier liant à sec et en milieu humide ;
(b) à prémélanger la vitamine D avec un second liant édulcorant dans une étape séparée ;
(c) à mélanger dans une autre étape séparée un diluant édulcorant, le reste dudit second liant édulcorant et un arôme avec les produits des étapes (a) et (b) tout en ajoutant un lubrifiant ;
(d) à comprimer éventuellement le mélange sur presse rotative.

## Claims

1. Therapeutic association of vitamin and calcium in unitary galenic tablet form, comprising as associated active ingredients calcium and at least one vitamin D, characterised in that, the galenic form being a tablet for biting, it further includes at least one first dry and moist binder combined in synergistic quantity with at least one diluant, at least one second binder, and at least one lubricant, at least one of said diluant and said second binder being a sweetening agent of the polyol type, and in that the ratio of calcium to vitamin D, expressed in mg of elemental Ca per IU of vitamin D, lies in the range 1 to 1.5.

2. Association according to claim 1, wherein the ratio of calcium to vitamin D, expressed in mg of elemental Ca per IU of vitamin D, lies in the range 1.2 to 1.3.

3. Association according to claim 1, wherein both said diluant and said second binder are sweetening agents of the polyol type.

4. Association according to claim 1 or 3, wherein said polyol(s) is (are) selected from mannitol, sorbitol, xylitol, and maltitol.

5. Association according to claim 1, wherein the calcium comes from a calcium salt selected from calcium carbonate, calcium pidolate, calcium lactate, calcium citrate, calcium gluconate, calcium chloride, calcium glucoheptonate, calcium glycerophosphate, and calcium phosphate.

6. Association according to claim 1, wherein the vitamin D is selected from vitamin D2 or ergocalciferol, vitamin D3 of cholecalciferol, or a mixture thereof.

7. Association according to claim 1, wherein said first dry and moist binder is selected from a cellulose, maltodextrin, and polyvinylpyrrolidone.

8. Association according to claim 1, wherein said lubricant is selected from magnesium stearate, stearic acid, hydrogenated castor oil, hydrogenated cotton oil and glycerol behenate.

9. Association according to claim 1, further containing a flavouring agent.

10. Association according to claim 1, further containing an acidifying agent.

11. Association according to claim 1, further containing an additional sweetening agent selected from sodium saccharinate, sodium cyclamate, and aspartam.

12. Association according to claims 2 and 3 taken in combination, with the following general formula :
| | |
|---|---|
| Calcium (carbonate) | 1250 mg |
| (quantity corresponding to elemental calcium | 500 mg) |
| Cholecalciferol | 4 mg * |
| Xylitol | 661 mg |
| Sorbitol | 500 mg |
| Polyvinylpyrrolidone | 45 mg |
| Flavouring (lemon, orange, etc.) | 20 mg |
| Magnesium stearate | 20 mg |
| | |
|---|---|
| *(vitamin D3 dosed at 100,000 IU/g) said formula corresponding to a finished tablet weighing 2500 mg | |

13. Method of obtaining a therapeutic association of vitamin and calcium according to any one of claims 1 to 12, characterised by consisting in:
a) granulating a calcium salt with a first wet and dry binder;
b) premixing the vitamin D with a second, sweetening binder in a separate step;
c) mixing in another separate step a sweetening diluant, an additional sweetening binder, and flavouring, with the products of steps a) and b), while also adding a lubricant; and
d) optionally compressing the mixture in a rotary press.

## Patentansprüche

1. Therapeutische Assoziation von Vitamin und Calcium in galenischer Einheitsform von Tabletten, die als aktive assoziierte Stoffe Calcium und zumindest ein Vitamin D aufweisen,
dadurch gekennzeichnet, daß
die galenische Form diejenige einer Kautablette ist, wobei sie außerdem zumindest ein erstes Bindemittel in trockenem und in feuchtem Milieu in synergetischer Menge mit zumindest einem Verdünnungsmittel kombiniert, zumindest ein zweites Bindemittel und zumindest ein Gleitmittel enthält, wobei zumindest das Verdünnungsmittel oder das zweite Bindemittel ein Süßstoff vom Typ Polyol ist, und dadurch, daß das Verhältnis von Calcium zu Vitamin D ausgedrückt in mg von elementarem Calcium pro internationaler Einheit von Vitamin D zwischen 1 und 1,5 beträgt.

2. Assoziation gemäß Anspruch 1, bei der das Verhältnis von Calcium in elementarer Form zu Vitamin D, ausgedrückt in mg von elementarem Calcium pro internationaler Einheit von Vitamin D, zwischen 1,2 und 1,3 beträgt.

3. Assoziation gemäß Anspruch 1, bei der das Verdünnungsmittel und das zweite Bindemittel beide Süßstoffe vom Typ Polyol.

4. Assoziation gemäß Anspruch 1 oder 3, bei der das (die) Polyol(e) aus Mannit, Sorbit, Xylit und Maltit ausgewählt ist (sind).

5. Assoziation gemäß Anspruch 1, bei der das Calcium von einem Calciumsalz stammt, das aus Calciumkarbonat, Calciumpidolat, Calciumlaktat, Calciumzitrat, Calciumchlorid, Calciumglucoheptonat, Calciumglycerophosphat und Calciumphosphat ausgewählt ist.

6. Assoziation gemäß Anspruch 1, bei der das Vitamin D aus Vitamin D2 oder Ergocalziferol, Vitamin D3 oder Cholecalziferol oder einer Mischung derselben ausgewählt ist.

7. Assoziation gemäß Anspruch 1, bei der das erste Bindemittel im trockenen und im feuchten Milieu aus Cellulose, Maltodextrin und Polyvinylpyrrolidon ausgewählt ist.

8. Assoziation gemäß Anspruch 1, bei der das Gleitmittel aus Magnesiumstearat, Stearinsäure, wasserstoffhaltigem Rizinusöl, wasserstoffhaltigem Cotonöl und Glycerinbehenat ausgewählt sind.

9. Assoziation gemäß Anspruch 1, die außerdem ein aromatisierendes Mittel enthält.

10. Assoziation gemäß Anspruch 1, die außerdem ein säurebildendes Mittel enthält.

11. Assoziation gemäß Anspruch 1, die außerdem einen zusätzlichen Süßstoff enthält, der aus Natriumsaccharin, Natriumcyclamat und Aspartam ausgewählt ist.

12. Assoziation gemäß den Ansprüchen 2 und 3 in Kombination, mit der allgemeinen Formel:
| | |
|---|---|
| Calcium (-carbonat) | 1 250mg |
| (entsprechende Menge an elementarem Calcium | 500 mg) |
| Cholecalciferol | 4mg* |
| Xylit | 661 mg |
| Sorbit | 500 mg |
| Polyvinylpyrrolidon | 45 mg |
| Aroma (Zitrone, Orange, usw.) | 20 mg |
| Magnesiumstearat | 20 mg |
| | |
|---|---|
| (* Vitamin D3 dosiert in 100 000 internationalen Einheiten/g, wobei die Formel einer fertigen Tablette mit 2 500 mg entspricht). | |

13. Verfahren zum Erhalten einer therapeutischen Assoziation von Vitamin und Calcium gemäß einem der Ansprüche 1 bis 12, dadurch gekennzeichnet, daß es darin besteht:
(a) ein Calciumsalz mit einem ersten Bindemittel im trockenen und im feuchten Milieu zu granulieren;
(b) das Vitamin D mit einem zweiten Süßstoff-Bindemittel in einem getrennten Schritt vorzumischen;
(c) in einem anderen getrennten Schritt ein Süßstoff-Verdünnungsmittel, den Rest des zweiten Süßstoff-Bindemittels und ein Aroma mit den Produkten der Schritte (a) und (b) zu mischen, wobei ein Gleitmittel hinzugefügt wird;
(d) eventuell das Gemisch in einer Rotationspresse zu komprimieren.
